# EUROPEAN PATENT SPECIFICATION

(11) **EP 1 063 961 B1**
(45) Date of publication and mention of the grant of the patent: **10.09.2003**
(21) Application number: 97943697.9
(22) Date of filing: 09.10.1997
(51) Int. Cl.: A61K 7/48

(54) **NON-SURGICAL METHOD FOR BREAST AUGMENTATION**
NICHT-CHIRURGISCHES VERFAHREN ZUR BRUSTVERGRÖSSERUNG
METHODE NON CHIRURGICALE D'AUGMENTATION MAMMAIRE

(43) Date of publication of application: 03.01.2001
(73) Proprietor: Giakoumakis, Marianthi, Montreal, Quebec H4G 3M2 (CA)
(72) Inventor: Giakoumakis, Marianthi, Montreal, Quebec H4G 3M2 (CA)
(74) Representative: Evans, Huw David Duncan
(86) International application number: CA9700744
(87) International publication number: WO98015258

(56) References cited:
- WO-A-85/00746
- US-A- 4 911 925
- US-A- 5 554 647
- DATABASE WPI Week 9711 Derwent Publications Ltd., London, GB; AN 97-117231 XP002052031 & RU 2 063 221 C (SINCHURINA) , 10 July 1996
- DATABASE WPI Week 8208, Derwent Publications Ltd., London, GB; AN 1982-15158E, XP002052032 & SU 822 824 A (MO NII KOSMETOLOGII) 23 April 1981
- H. Wagner, "Pharmazeutische Biologie, Drogen und ihre Inhaltsstoffe", Fourth Edition, Gustav Fischer Verlag, Stuttgart, New York, 1988, p. 359

## Description

### TECHNICAL FIELD:

The present invention relates to a method for breast augmentation, and in particular to a non-surgical method for breast augmentation.

### BACKGROUND ART:

Breast augmentation is a growing industry. In the United States alone, it is estimated that each year over one million women undergo surgery for breast enlargement.

The most commonly used procedure for breast augmentation is mammaplastry, which is, surgical augmentation of the breasts. This procedure generally involves making a surgical incision to create a pocket in the breast followed by insertion of a mammary prosthesis.

Present day prosthesis are usually made from a polysilicone elastomer sac filled with a silicone gel or mixture thereof. However, such implants are not entirely safe. They have been know to leak and rupture, causing long-term adverse effects on the body.

Saline-filled implants are also popular. They are considered safer because saline solution is similar to other body fluids and leakage thereof will simply be absorbed without harm. However, saline-filled implants do not feel or look as natural as gel implants. They tend to wrinkle or ripple under the skin preventing the breast from moving and hanging naturally. Moreover, it is believed that saline implants have a higher rate of leaking and deflation than silicone gel implants. Thus frequent surgery may be required to replace the implants.

Because of all of the problems associated with implants, alternatives have been sought. The latest in implant alternatives is a new technique known by the acronym BAM∼3I (bilateral augmentation mammoplastry by injection). The BAMBI method involves surgery and entails liposuctioning fat from the body, for example from the buttocks or thighs for injection into the chest. Although some plastic surgeons believe the method is safe, others do not. The surgery may cause dangerous infection, and the fat can calcify resulting in masses of scar tissue which can mimic or mask breast cancer. This, in turn, can lead to unnecessary biopsies if there is no cancer, or delay detection if there is cancer.

More natural, non-intrusive methods have also been sought. For example, it has been found that cocoa butter augments breast size. The use of cocoa butter for breast augmentation is mentioned on page 285 of the Edgar Cayce Handbook for Health through Drugless Therapy. Cayce states, in his reading numbered 934-13, that a method to enlarge the breast is obtained, as follows: "This is a little bit late in beginning [the woman was aged thirty-two) but if there is the massage of the mammary glands with cocoa butter-not on the breast itself, but under the arm and lower and in the area between the breast-you can get 'em as big or as little as you wish". However, it had been found by the applicant that augmentation of the breast using Cayce's method alone did not last, i.e. the breast does not remain enlarged with the application of cocoa butter for long and eventually returns to the same size, as prior to the application of cocoa butter, in the following few days.

WO85/00746 discloses skin care and shaving compositions comprising Vitamin E and cocoa butter, whereby the latter exhibits a greater skin penetration when warm.

Database WPI Week 8208 Derwent Publications Ltd., London, GB; AN82-15158E XP002052032 & SU 822 824 B discloses a face cream comprising cocoa butter and Vitamin E for preventing skin ageing and improving the turgor, smoothness and elasticity of the skin. Database WPI Week 9711 Derwent Publications Ltd., London, GB; AN97-117231 XP002052031 & RU 2 063 221 C discloses a nourishing face cream composition comprising cocoa butter and an oil solution of Vitamin E.

US 4 911 925 discloses a skin treatment substance for treatment of ageing skin to increase its breathing ability, regulate the oxidative process in skin, positively effect blood circulation in the skin and improve the regenerative ability and activate metabolism in skin, comprising cocoa butter and corn oils, which according to *H.Wagener "Pharmazeutische Biologie, Drogen und ihre Inhaltsstoffe", Fourth Edition, Gustav Fischer Verlag, Stuttgart, New York, 1988, page 359,* are known to comprise Vitamin E.

### DISCLOSURE OF THE INVENTION

An object of the present invention is to provide a non-surgical method for breast size augmentation and to sustain the augmented breast size.

In accordance with one aspect of the present invention there is provided a method for cosmetically augmenting the size of a human breast and for sustaining the augmented breast size, the method comprising applying cocoa butter to the breast so as to augment the size of the breast and applying Vitamin E to the breast so as to sustain the augmented breast size.

In accordance with another aspect of the present invention there is provided the use of cocoa butter and Vitamin E for cosmetically augmenting the size of a human breast and for sustaining the augmented breast size.

In accordance with another aspect of the invention there is provided The use of cocoa butter and Vitamin E for the manufacture of a composition for cosmetically augmenting the breast size of a human and for sustaining the augmented breast size.

Preferably, the cocoa butter is applied in an amount sufficient to achieve the desired augmentation of the breast and the Vitamin E is applied in an amount sufficient to sustain the desired augmented breast size.

In one embodiment of the invention, the cocoa butter and Vitamin E are simultaneously applied to the breast.

In another embodiment of the invention the cocoa butter is first applied to the breast, and the Vitamin E is applied subsequently.

Preferably, the proportion of cocoa butter to Vitamin E is 18 :1.

The advantages of the present invention are that there are no surgical procedures necessary, hence eliminating all the potential problems, either physical, emotional or psychological, that might come with having surgery. Therefore, the present invention provides a safer and more natural method than surgery for enlarging the size of the human breast and sustaining the augmented breast size. Moreover, the method is simple and inexpensive. In addition, there is the benefit derived from the psychological satisfaction and emotional well being women would experience from augmenting their breast size by using this healthier and more natural invention than surgical breast augmentation. Therefore, this method is an advance to man kind because it is a more natural and safer method of breast augmentation than breast augmentation attained by surgery.

### MODES FOR CARRYING OUT THE INVENTION:

The preferred method of the present invention includes applying cocoa butter (a fatty substance obtained from cacao seeds), for example Derma-E*, and Vitamin E, for example d-alpha-tocopherol acetate by Jamieson Natural Source*, to the breast for enlargement thereof. The cocoa butter and Vitamin E can be applied separately or as a mixture. The breast includes an area generally between the second and seventh rib and extending between the axilla (armpit) and the sternum. Further to Mr. Cayce's method, it has been found by the applicant that the application of the cocoa butter also on the globe of the breast itself promotes enlargement thereof. It is the applicant's belief that the cocoa butter is absorbed into the breast, hence enlarging the size of the breast by the breast assimilating the cocoa butter and storing the cocoa butter around the gland lobules of the mammary gland connected to the breast therein, by means of the breast assimilating the cocoa butter as fat. Therefore, further to Mr. Cayce's method, it is desirable to apply cocoa butter also to the globe of the breast in order to augment the size of the breast. Preferably, the application of cocoa butter includes gentle rubbing on the skin of the breast, that is the area of application, to facilitate the absorption of the cocoa butter into the breast to augment the breast in size. Furthermore, in the application of cocoa butter or Vitamin E it is preferable to apply cocoa butter or Vitamin E in thin layers at a time to create a friction to the skin that lessens the amount of time needed for the absorption of cocoa butter or vitamin E into the breast. Also, first heating the breast area before the application of cocoa butter or vitamin E lessens the amount of time needed for the absorption of either ingredient into the breast.
Trade-marks

The amount of cocoa butter used is directly proportional to the augmentation of the breast's size. It has been found that approximately 50g of cocoa butter per breast enlarges each breast from an A size bra cup to a B size bra cup. For such an amount, the application time for the applicant, at a younger age of approximately 30 years old and under, was approximately 11 consecutive hours of application of the cocoa butter. This was before the applicant's discovery that Vitamin E would sustain the augmented breast size, and so the breast would reduce to the same breast size as before the application of the cocoa butter. However, at an older age, approximately the age of thirty and over, the applicant had found that response time of the body to the application of cocoa butter took significantly longer for the breast to augment in size. At the age of 35 years, it would then take the applicant 4 to 6 hours to absorb about 7g of cocoa butter.

Following the application of the cocoa butter, Vitamin E is applied to the breast. Further to Cayce's statement, it has been found that the use of Vitamin E sustains the augmented breast size. Vitamin E sustains the size of the breast augmented by the application of cocoa butter to the breast. It had been found by the applicant that the omission of the use of Vitamin E caused the body to lose all the augmentation in breast size derived from the application of the cocoa butter in the days that followed the application of the cocoa butter.

It is the applicant's experience at the age of 35 that the application of approximately 400mg of Vitamin E was satisfactory to sustain the breast size augmented by the application to the breast and absorption by the breast, of approximately 7g of cocoa butter. Therefore, giving a proportion of cocoa butter to Vitamin E of about 18:1. It has been found by the applicant that subsequent applications of cocoa butter and Vitamin E to the breast further augmented and sustained the breast in size and that at no time did the augmented breast size reduce again in size from one application sitting to the next. It has been found that simply smearing the Vitamin E to the breast area without the use of gentle rubbing to aid in the absorption of the Vitamin E is also sufficient to sustain the size of the breast augmented by the application of cocoa butter.

Also the application of Vitamin E does not necessarily need to be applied just after the application of cocoa butter for this method to work. Even the application of Vitamin E to the breast prior to the application of the cocoa butter has also been found by the applicant to have the desired effect in sustaining the breast size augmented by the application of cocoa butter.

Augmentation of the size of the breast and sustaining the augmented size can also be achieved using a composition of cocoa butter and Vitamin E in an approximate proportion of 18 to 1. the composition is produced by melting the cocoa butter at very low heat, approximately 40°C, and the Vitamin E is added thereto to first obtain a mixture. the mixture was stirred for 30 to 40 seconds and let stand to cool before use to obtain the final composition.

It will be recognized by one skilled in the art that the above mentioned amounts, quantity and proportion of ingredients of cocoa butter and Vitamin E are merely guidelines. The exact amounts and proportions of cocoa butter and Vitamin E necessary to augment the breast size and sustain the augmented breast size can vary from one person to another, depending on, for example, different body types and different body compositions, and the person's age.

## Claims

1. A method for cosmetically augmenting the size of a human breast and for sustaining the augmented breast size, the method comprising applying cocoa butter to the breast so as to augment the size of the breast and applying Vitamin E to the breast so as to sustain the augmented breast size.

2. A method as claimed in claim 1, wherein the cocoa butter is applied in an amount sufficient to achieve the desired augmentation of the breast and the Vitamin E is applied in an amount sufficient to sustain the desired augmented breast size.

3. A method as claimed in claim 1, wherein the cocoa butter and Vitamin E are simultaneously applied to the breast.

4. A method as claimed in claim 1, wherein the cocoa butter is first applied to the breast, and the Vitamin E is applied subsequently.

5. A method as claimed in any preceding claim, wherein the proportion of cocoa butter to Vitamin E is 18:1.

6. A method as claimed in any preceding claim, wherein 7g of cocoa butter and 400mg of Vitamin E are applied to the breast.

7. The use of cocoa butter and Vitamin E for cosmetically augmenting the size of a human breast and for sustaining the augmented breast size.

8. The use as claimed in claim 7, wherein the proportion of cocoa butter to Vitamin E is 18:1.

9. The use of cocoa butter and Vitamin E for the manufacture of a composition for cosmetically augmenting the breast size of a human and for sustaining the augmented breast size.

## Patentansprüche

1. Ein Verfahren zur kosmetischen Vergrößerung der menschlichen Brust und zur Aufrechterhaltung der vergrößerten Brustgröße, wobei das Verfahren darin besteht, Kakaobutter auf die Brust aufzutragen, wodurch die Brust vergrößert wird, und Vitamin E auf die Brust aufzutragen, wodurch die vergrößerten Brustgröße aufrechterhalten wird.

2. Ein Verfahren nach Anspruch 1, wobei die Menge der aufgetragenen Kakaobutter für die erwünschte Brustvergrößerung ausreicht, und die Menge des aufgetragenen Vitamins E dazu ausreicht, die erwünschte vergrößerte Brustgröße aufrechtzuerhalten.

3. Ein Verfahren nach Anspruch 1, wobei die Kakaobutter und das Vitamin E gleichzeitig auf die Brust aufgetragen werden.

4. Ein Verfahren nach Anspruch 1, wobei zuerst die Kakaobutter auf die Brust aufgetragen wird und anschließend das Vitamin E aufgetragen wird.

5. Ein Verfahren nach einem der vorhergehenden Ansprüche, wobei die Kakaobutter zum Vitamin E im quantitativen Verhältnis 18:1 steht.

6. Ein Verfahren nach einem der vorhergehenden Ansprüche, wobei 7 g Kakaobutter und 400 mg Vitamin E auf die Brust aufgetragen werden.

7. Die Verwendung von Kakaobutter und Vitamin E zur kosmetischen Vergrößerung der menschlichen Brust und zur Aufrechterhaltung der vergrößerten Brustgröße.

8. Die Verwendung nach Anspruch 7, wobei die Kakaobutter zum Vitamin E im quantitativen Verhältnis 18:1 steht.

9. Die Verwendung von Kakaobutter und Vitamin E zur Herstellung einer Mischung für die kosmetische Vergrößerung der menschlichen Brust und für die Aufrechterhaltung der vergrößerten Brustgröße.

## Revendications

1. Procédé pour augmenter de manière cosmétique la taille d'un sein et pour maintenir la taille plus grande du sein, le procédé consistant à appliquer du beurre de cacao sur le sein de manière à augmenter la taille du sein et aussi à appliquer de la vitamine E sur le sein de manière à maintenir la taille plus grande du sein.

2. Procédé, selon la revendication 1, **caractérisé en ce que** le beurre de cacao est appliqué en quantité suffisante pour réaliser l'augmentation voulue de la taille du sein, et **caractérisé en ce que** la vitamine E est appliquée en quantité suffisante pour maintenir la taille plus grande du sein.

3. Procédé, selon la revendication 1, **caractérisé en ce que** le beurre de cacao et la vitamine E sont appliqués en même temps sur le sein.

4. Procédé, selon la revendication 1, **caractérisé en ce que** le beurre de cacao est appliqué en premier sur le sein, et la vitamine E est appliquée par la suite sur le sein.

5. Procédé, selon l'une quelconque des revendications précédentes, **caractérisé en ce que** la proportion entre le beurre de cacao et la vitamine E est de 18:1.

6. Procédé, selon l'une quelconque des revendications précédentes, **caractérisé en ce que** 7g de beurre de cacao et 400mg de vitamine E sont appliqués sur le sein.

7. Utilisation de beurre de cacao et de vitamine E pour augmenter de manière cosmétique la taille d'un sein et pour maintenir la taille plus grande du sein.

8. Utilisation, selon la revendication 1, **caractérisée en ce que** la proportion entre le beurre de cacao et la vitamine E est de 18:1.

9. Utilisation de beurre de cacao et de vitamine E pour fabriquer une composition servant à augmenter de manière cosmétique la taille d'un sein et pour maintenir la taille plus grande du sein.
